(19) Europäisches Patentamt
European Patent Office
Office européen des brevets

(11) EP 1 449 829 B1

(12) EUROPEAN PATENT SPECIFICATION

(45) Date of publication and mention of the grant of the patent:
**28.10.2009 Bulletin 2009/44**

(51) Int Cl.:
***C07C 303/44*** (2006.01) ***C07C 311/37*** (2006.01)

(21) Application number: **04250684.0**

(22) Date of filing: **09.02.2004**

# (54) Process for preparing Tamsulosin hydrochloride

Verfahren zur Herstellung von Tamsulosin Hydrochlorid

Procédé de preparation du chlorhydrate de Tamsulosine

(84) Designated Contracting States:
**CH DE ES FR GB IT LI**

(30) Priority: **12.02.2003 KR 2003008871**

(43) Date of publication of application:
**25.08.2004 Bulletin 2004/35**

(73) Proprietor: **BORYUNG PHARMACEUTICAL CO., LTD.**
**Seoul 110-450 (KR)**

(72) Inventors:
- **Kim, Sang-Lin,**
  **4-1008 Dongshin Apt**
  **Seoul 139-909 (KR)**
- **Kim, Ji-han,**
  **Seoul 150-823 (KR)**
- **Lee, Joon.kwang,**
  **201 Taeyang Mansion**
  **Kwangmyung-shi,**
  **Kyunggi-do 423-814 (KR)**
- **Sun, Yong-ho**
  **Seoul 151-801 (KR)**
- **Han, Nam-suk**
  **Ansan-shi,**
  **Kyunggi-do 425-180 (KR)**
- **Lee, Seung-ho,**
  **104-1409 Yeoksam Lucky Apt.**
  **Seoul 135-858 (KR)**
- **Kim, Mi-soon,**
  **Daewoo Apt. Na-205**
  **Siheung-si,**
  **Gyunggi-do (KR)**
- **Lee, Jung-eun**
  **Daerim epyunhansesang Apt. 101-1703**
  **Anyang-si**
  **Gyunggi-do (KR)**

(74) Representative: **Browne, Robin Forsythe**
**Leaman Browne Limited**
**Pearl Chambers**
**22 East Parade**
**Leeds, Yorkshire LS1 5BY (GB)**

(56) References cited:
**AT-B- 397 960** **US-A- 4 373 106**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 449 829 B1

## Description

The present invention relates to a process for preparing tamsulosin hydrochloride, and more particularly to a process for preparing tamsulosin hydrochloride by adding a carboxylic compound to tamsulosin in an organic solvent to obtain tamsulosin carboxylate in the form of precipitates and adding hydrochloric acid to the precipitated tamsulosin carboxylate to prepare highly pure tamsulosin hydrochloride containing only a trace amount of impurities.

Description of the Related Art

Tamsulosin ((R)-5-{2-[2-(2-ethoxyphenoxy)ethyl]amino} propyl-2-methoxybenzenesulfonamide) is a compound represented by Formula 1 below:

Formula 1

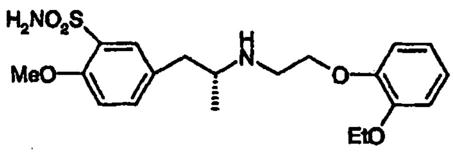

It is known that tamsulosin exhibits α-adrenergic blocking actions and is useful as a therapeutic agent for benign prostatic hypertrophy (BPH), hypertension and congestive heart failure.

Since the discovery of the usefulness of tamsulosin, a number of studies on methods for preparing tamsulosin are in the making and various proposals have been made in e.g., U.S. Patent Nos. 4,217,305 and 4,373,106. According to these patents, tamsulosin is prepared from a mixture of the racemic compounds as a raw material. In addition, Korean Patent Publication No. 94-7746 discloses a process for preparing tamsulosin as an optically active substance using a chiral amine.

However, since the prior art processes involve the separation of one of the isomers from the racemic mixture, they have problems of a low yield (< 50%) or undesirable side reactions, which causes an extremely low yield.

Thus, the present inventors have developed a process for preparing tamsulosin of Formula 1 in high yield, and filed a patent applicationonMay7, 2001 (KoreanPatentLaid-openNo. 2002-0085278, Unexamined Pub. date: Nov. 16, 2002).

According to this patent application, as depicted in Reaction Scheme 1 below, hydrogen gas is first fed to a mixture of an aziridine compound of Formula 2 and Pd/C to perform a reduction reaction. Thereafter, the reduction product is recrystallized from isopropanol, and then a mixture of HCl (in isopropanol) is added thereto in a mixed solvent (methanol/isopropanol) to yield tamsulosin hydrochloride of Formula 1. Since this process does not accompany any formation of racemic compounds and there are few or no side reactions, it enables the preparation of tamsulosin in high yield, compared to conventional preparation processes.

Reaction Scheme 1

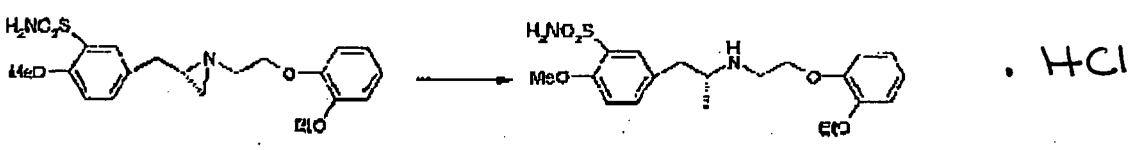

(2)

However, since the tamsulosin hydrochloride thus prepared contains small amounts of impurities, such as unreacted reactants and side-products, there are still problems of low purity. There remains a need in the art for processes for preparing high purity tamsulosin in high yield and in a simple manner.

AT-B-397960 discloses acid addition salts tamsulosin and that these can be purified by a reprecipitation process. US-A-4373106 discloses that recrystallisation of tamsulosin in the form of precipitated acid addition salts provides a highly pure product.

SUMMARY OF THE INVENTION

Therefore, the present invention has been made in view of the above problems, and it is an object of the present invention to provide a process for purifying tamsulosin in high yield and in a simple manner.

In order to accomplish the above object of the present invention, there is provided a process for preparing tamsulosin hydrochloride comprising the steps of:

a) adding a carboxylic compound to tamsulosin represented by Formula 1 below:

Formula 1

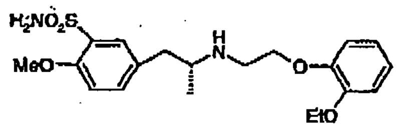

in an organic solvent to obtain a tamsulosin carboxylate represented by Formula 3 below:

Formula 3

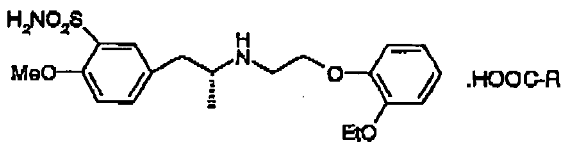

wherein

R is an alkyl, aryl, allyl, vinyl or benzyl group in the form of a precipitate;

b) dissolving the precipitated tamsulosin carboxylate in a solvent selected from the group consisting of: an alcohol, water and mixtures thereof; and

c) adding hydrochloric acid to the tamsulosin carboxylate to prepare tamsulosin hydrochloride represented by Formula 4 below:

Formula 4

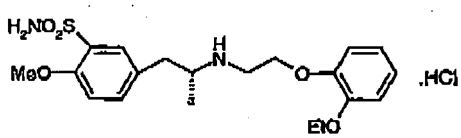

According to the process of the present invention, the tamsulosin hydrochloride is prepared with a purity of 99.0% or higher, and impurities arc present in an amount of 0.3% or less. This will be apparent from the example and tests example which follow. In conclusion, the process of the present invention enables the preparation of tamsulosin of a high purity in the form of a pharmaceutically acceptable hydrochloride salt in high yield and in a simple manner.

As the organic solvent that can be used in the process of the present invention, all solvents usable for the purification of common compounds can be used. Specific examples of the organic solvent include, but are not limited to isopropanol, methanol, ethanol and mixtures thereof The organic solvent is added to further increase the purity of the tamsulosin hydrochloride as the final product.

As the carboxylic compound that can be used in the process of the present invention, all common carboxylic compounds can be used. Particularly, the carboxylic compound is preferably selected from the group consisting of mandelic acid, salicylic acid, maleic acid, malonic acid, malic acid, anisic acid and mixtures thereof. Among them, mandelic acid is more preferred. The carboxylic compound is added to separate the tamsulosin carboxylate as the intermediate product in higher yield. This separation enables the preparation of high purity tamsulosin hydrochloride as the final product.

DESCRIPTION OF THE PREFERRED EMBODIMENTS

The present invention will be explained in more detail below by one embodiment of a process for purifying tamsulosin according to the present invention.

First, an organic solvent and a carboxylic compound are added to tamsulosin of Formula 1 to obtain tamsulosin carboxylate. At this step, tamsulosin of Formula I can be prepared in accordance with a conventional process, e.g., the process described in Korean Patent Laid-open No. 2002-0085278. The organic solvent is preferably selected from the group consisting of ethylacetate, acetone, chloroform, isopropanol, methanol, ethanol and mixtures thereof. As the carboxylic compound, at least one compound selected from the group consisting of mandelic acid, salicylic acid, maleic acid, malonic acid, malic acid and anisic acid, can be preferably used. Among them mandelic acid is most preferred.

The combined use of the organic solvent and the carboxylic compound enables of the separation of the tamsulosin carboxylate as the intermediate product in higher yield. This separation facilitates the preparation of high purity tamsulosin hydrochloride as the final product.

The tamsulosin carboxylate is obtained in the form of a precipitate- After the tamsulosin carboxylate is recovered by filtration, it is dissolved in a solvent under heating. Hydrochloric acid is added to the resulting solution to prepare tamsulosin Hydrochloride as the final product. At this step, an alcohol such as isopropanol, methanol or ethanol, water, r a mixture thereof is used as the solvent.

According to the process of the present invention, the tamsulosin hydrochloride is prepared with a high purity of 99.0% or higher, and impurities are present in an amount of 0.3% or less. In conclusion, the process of the present invention enables the preparation of tamsulosin with a high purity in the form of a pharmaceutically acceptable hydrochloride in high yield and in a simple manner.

Reference example 1: Preparation of tamsulosin (see, Preparative Example 6 of Korean Patent Laid-open No. 2002-0085278)

First, hydrogen gas was fed into a mixture of 33g of an aziridinc compound of Formula 2, 660ml of methanol and 3.3g of Pd/C with stirring at 45~50°C for 12 hours. After the reaction mixture was filtered through Celite and concentrated, 65ml of saturated sodium bicarbonate solution was added thereto. The resulting mixtures was extracted with 130ml and 50ml of chloroform. The obtained organic layer was dried over anhydrous sodium sulphate, filtered and concentrated. The concentrate was added to 330ml of isopropanol, dissolved under heating, cooled, filtered and dried to yield 26.5g of tamsulosin ((R)-5-{2-[2-(2-ethoxyphenoxy)ethyl]amino}propyl-2-methoxyben zenesulfonamide) of Formula 1 (Impurity content: 4.08%).

Comparative Example: Preparation of tamsulosin hydrochloride (see, Preparative Example 6 of Korean Patent Laid-open No. 2002-0085278)

After 13.0g of the tamsulosin prepared in the Reference Example above was dissolved in a mixed solvent of 110ml of methanol and 150ml of isopropanol under heating, 20ml of 4.5N HCl (in isopropanol) was slowly added to the solution. The reaction mixture was cooled, filtered and dried to yield 13. 4g of tamsulosin hydrochloride (Impurity content: 0.63%)

Example: Preparation of tamsulosin hydrochloride

260ml of ethylacetate and 5.8g of mandelic acid were added to 13.0g of the tamsulosin prepared in the Reference Example above. The resultant mixture was refluxed under stirring for 1 hour, cooled, filtered and dried to obtain 16.5g of tamsulosin mandelate in the form of precipitates. The tamsulosin mandelate was dissolved in a mixed solvent of 65ml of methanol and 80ml of isopropanol under heating, and then 20ml of 4.5N HCl (in isopropanol) was slowly added thereto. The resulting mixture was cooled, filtered and dried to yield 12.9g of high purity tamsulosin hydrochloride (Impurity content: 0.25%)

Test Example: Purity measurement of tamsulosin

The purity of the tamsulosin hydrochloride prepared in the Example above was measured in accordance with

quantitative methods using HPLC and potentiometric titration. Operating conditions of the quantitative methods and the measurement results are set forth as below:

(1) HPLC conditions

1) Preparation of test solution

0.05g of the tamsulosin hydrochloride prepared in the Example above was dissolved in 10ml of the mobile phase obtained in Condition 1 below to prepare a test solution.

2) Preparation of standard solution

The mobile phase obtained in Condition 1 below was added to 2.0ml of the test solution until the mixture was made up to a total volume of 50ml. To 2.5ml of the mixture was further added the mobile phase obtained in Condition 1 below until the resulting mixture was made up to a total volume of 50ml to prepare a standard solution.

3) Condition 1

* Detector: Ultraviolet absorption spectrometer (Detection wavelength: 225nm)
* Column: 4mm x 15cm, 5μm Cosmosil $C_{18}$ (ODS)
* Column Temperature: A constant temperature around 40°C
* Choice of Column: A mixture of 5.0mg of tamsulosin hydrochloride and 0.01g of propyl paraoxybenzoate were added to the mobile phase until the total volume was 20ml, and then the resulting mixture was dissolved. 2.0ml of the solution was added to another mobile phase until the total volume was 20ml. When 10μl of the solution was operated in accordance with Condition 1, the tamsulosin and the propyl paraoxybenzoate was sequentially separated from the solution and the degree of separation was 12 or higher.
* Mobile phase: After 8.7ml of perchloric acid and 3.0g of sodium hydroxide were dissolved in 1,900ml of water, pH of the solution was controlled to 2.0 using sodium hydroxide solution. Water was added to the solution until the total volume was 2, 000ml, and then 600ml of acetonitrile was added thereto to prepare a mobile phase.
* Flow rate: The retention timeof tamsulosin was controlled to about 6 minutes.
* Detection sensitivity: The highest level of tamsulosin measured in 10μl of the standard solution was controlled so as to be about 10% of the full scale.
* Measurement range: Until the elution of tamsulosin was completed (except solvent peaks)
* Feeding amount: 10μl

4) Condition 2

* Detector: Ultraviolet absorption spectrometer (Detection wavelength: 225nm)
* Column: 4mm x 15cm, 5μm Cosmosil $C_{18}$ (ODS)
* Column Temperature: A constant temperature around 40ºC
* Choice of Column: As defined in Condition 1 above.
* Mobile phase: After 8.7ml of perchloric acid and 3.0g of sodium hydroxide were dissolved in 1,900ml of water, pH of the solution was controlled to 2.0 using sodium hydroxide solution. Water was added to the solution until the total volume was 2, 000ml, and then 1,428ml of acetonitrile was added thereto to prepare a mobile phase.
* Flow rate: The retention timeof tamsulosin was controlled to about 2.5 minutes.
* Detection sensitivity: The peak height of tamsulosin measured in 10μl of the standard solution was controlled so as to be about 20% of the full scale.
* Measurement range: Within 5-fold of the retention time of tamsulosin after the peak of tamsulosin.
* Feeding amount: 10μl

5) Operation and Calculation:

The test solution and the standard solution were tested in accordance with the standard test method for liquid chromatography of the Korea Pharmacopedia under the above conditions.

* Calculation:

$$\text{Total amount of impurities (\%)} = 0.2 \times (A_{T1}/A_{s1} + A_{T2}/A_{s2})$$

where $A_{T1}$ is the total area under all peaks obtained before the elution of tamsulosin (in test solution) dissolved in the mobile phase of Condition 1 above,
$A_{S1}$ is the area under the peak of tamsulosin (in standard solution) dissolved in the mobile phase of Condition 1 above,
$A_{T2}$ is the total area under all peaks obtained after the elution of tamsulosin (in test solution) dissolved in the mobile phase of Condition 2 above, and
$A_{S2}$ is the area under the peak of tamsulosin (in standard solution) dissolved in the mobile phase of Condition 2 above.

(2) Quantitative method

About 0.7g of dried tamsulosin hydrochloride was dissolved in 5.0ml of formic acid. Immediately after 75ml of a mixture of glacial acetic acid and anhydrous acetic acid (3:2) was added to the solution, titration using 0.1N perchloric acid was performed (potentiometric titration).

At this time, a blank test was conducted for calibration. 1.0ml of 0.1N perchloric acid = 44.5mg of $C_{20}H_{23}N_2O_5S \cdot HCl$

(3) HPLC analytical results

Asaresultofanalyzingthetamsulosinhydrochlorideprepared in the Example above under the conditions defined above, it was found that only a small amount (0.3%) of impurities was contained in the tamsulosin hydrochloride.

(4) Quantitative method (potentiometric titration)

As a result of quantifying the tamsulosin hydrochloride prepared in the Example above in accordance with the procedure (2) described above, it was demonstrated that the tamsulosin hydrochloride was present in an amount of 99.0% or more.

As apparent from the above description, according to the process of the present invention, a carboxylic compound is added to the tamsulosin to obtain a tamsulosin carboxylate, and then hydrochloric acid is added to the tamsulosin carboxylate to prepare tamsulosin hydrochloride. The tamsulosin hydrochloride is prepared with a high purity of 99.0% or higher, and impurities are present in an amount of 0.3% or less. In addition, the process of the present invention enables the preparation of tamsulosin of high purity in the form of a pharmaceutically acceptable hydrochloride in a simple manner. Furthermore, since the process of the present invention enables the purification of tamsulosin in high yield, it is economically efficient.

## Claims

1. A process for preparing tamsulosin hydrochloride comprising the steps of:

    a) adding a carboxylic compound to tamsulosin represented by Formula 1 below:

    Formula 1

    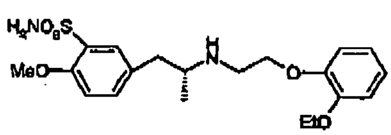

    in an organic solvent to obtain a tamsulosin carboxylate represented by Formula 3 below:

Formula 3

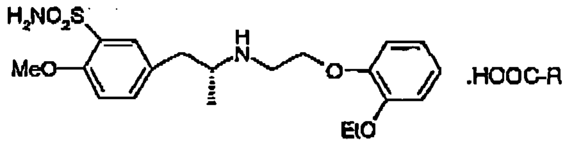

wherein

R is an alkyl, aryl, allyl, vinyl or benzyl group in the form of a precipitate;

b) dissolving the precipitated tamsulosin carboxylate in a solvent selected from the group consisting of: an alcohol, water and mixtures thereof; and

c) adding hydrochloric acid to the tamsulosin carboxylate to prepare tamsulosin hydrochloride represented by Formula 4 below:

Formula 4

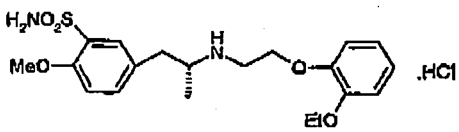

**2.** The process according to claim 1, wherein the organic solvent of step a) is at least one solvent selected from the group consisting of: ethylacetate, acetone, chloroform, isopropanol, methanol and ethanol.

**3.** The process according to claim 1 or 2, wherein the carboxylic compound is at least one acid selected from the group consisting of: mandelic acid, salicyclic acid, maleic acid, malonic acid, malic acid and anisic acid.

**4.** The process of claim 3, wherein the carboxylic acid is mandelic acid.

**5.** The process according to any preceding claim, wherein the alcohol of step b) is at least one solvent selected from the group consisting of isopropanol, ethanol and methanol.

**6.** The process according to any preceding claim including the step of filtering the tamsulosin hydrochloride.

**Patentansprüche**

**1.** Verfahren zur Herstellung von Tamsulosinhydrochlorid, das die folgenden Schritte umfasst:

a) Zugabe einer Carboxylverbindung zu Tamsulosin, das durch untenstehende Formel 1 dargestellt ist:

Formel 1

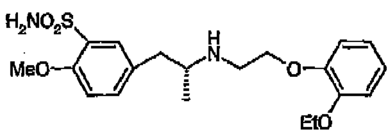

in einem organischen Lösungsmittel, um Tamsulosincarboxylat zu erhalten, das durch untenstehende Formel

3 dargestellt ist:

Formel 3

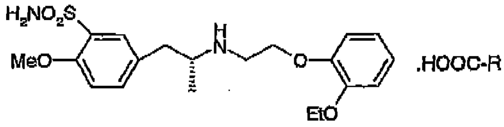

wobei

R eine Alkyl-, Aryl-, Allyl-, Vinyl- oder Benzylgruppe ist, in der Form eines Präzipitates;

b) Auflösen des präzipitierten Tamsulosincarboxylat in einem Lösungsmittel ausgewählt aus der Gruppe bestehend aus: einem Alkohol, Wasser und Mischungen daraus; und
c) Zugabe von Salzsäure zu dem Tamsulosincarboxylat zur Herstellung von Tamsulosinhydrochlorid, dargestellt durch untenstehende Formel 4:

Formel 4

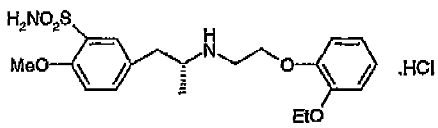

2. Das Verfahren gemäß Anspruch 1, wobei das organische Lösungsmittel von Schritt a) mindestens ein Lösungsmittel ist, das ausgewählt ist aus der Gruppe bestehend aus: Ethylacetat, Aceton, Chloroform, Isopropanol, Methanol und Ethanol.

3. Das Verfahren gemäß Anspruch 1 oder 2, wobei die Carboxylverbindung mindestens eine Säure ist, die ausgewählt ist aus der Gruppe bestehend aus: Mandelsäure, Salicylsäure, Maleinsäure, Malonsäure, Apfelsäure und Anissäure.

4. Das Verfahren gemäß Anspruch 3, wobei die Carboxylsäure Mandelsäure ist.

5. Das Verfahren gemäß irgendeinem der vorhergehenden Ansprüche, wobei der Alkohol von Schritt b) mindestens ein Lösungsmittel ist, das ausgewählt ist aus der Gruppe bestehend aus Isopropanol, Ethanol und Methanol.

6. Das Verfahren gemäß irgendeinem der vorhergehenden Ansprüche, das den Schritt der Filterung von Tamsulosinhydrochlorid einschließt.

## Revendications

1. Procédé de préparation d'hydrochlorure de tamsulosine comprenant les étapes suivantes :

a) ajout d'un composé carboxylique à la tamsulosine représentée par la Formule 1 ci-dessous :

Formule 1

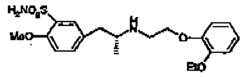

dans un solvant organique afin d'obtenir un carboxylate de tamsulosine représenté par la Formule 3 ci-dessous :

Formule 3

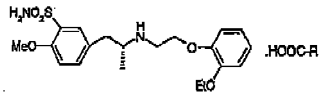

dans lequel

R est un groupe alkyle, aryle, allyle, vinyle ou benzyle sous la forme d'un précipité ;

b) dissolution du carboxylate de tamsulosine précipité dans un solvant sélectionné dans le groupe constitué d'un alcool, d'eau et de mélanges de ceux-ci ; et
c) ajout d'acide chlorhydrique au carboxylate de tamsulosine pour préparer l'hydrochlorure de tamsulosine représenté par la Formule 4 ci-dessous :

Formule 4

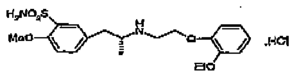

2. Procédé selon la revendication 1, dans lequel le solvant organique de l'étape a) est au moins un solvant sélectionné dans le groupe constitué de l'acétate d'éthyle, de l'acétone, du chloroforme, de l'isopropanol, du méthanol et de l'éthanol.

3. Procédé selon la revendication 1 ou 2, dans lequel le composé carboxylique est au moins un acide sélectionné dans le groupe constitué de l'acide mandélique, de l'acide salicylique, de l'acide maléique, de l'acide malonique, de l'acide malique et de l'acide anisique.

4. Procédé de la revendication 3, dans lequel l'acide carboxylique est l'acide mandélique.

5. Procédé selon l'une des revendications précédentes, dans lequel l'alcool de l'étape b) est au moins un solvant sélectionné dans le groupe constitué de l'isopropanol, de l'éthanol et du méthanol.

6. Procédé selon l'une des revendications précédentes comprenant l'étape de filtrage de l'hydrochlorure de tamsulosine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4217305 A
- US 4373106 A
- KR 947746
- KR 20020085278
- AT 397960 B